(19) Europäisches Patentamt
European Patent Office
Office européen des brevets

(11) Numéro de publication : **0 380 411 B1**

(12) # FASCICULE DE BREVET EUROPEEN

(45) Date de publication du fascicule du brevet : **11.11.92 Bulletin 92/46**

(51) Int. Cl.$^5$ : **C07C 69/33,** C07C 67/29, C07C 69/708

(21) Numéro de dépôt : **90400198.9**

(22) Date de dépôt : **24.01.90**

(54) **Procédé de préparation de tensio-actifs non-ioniques à partir de l'isopropylidène-1,2 époxypropyl-3 glycérol et d'un acide carboxylique, nouveaux tensio-actifs non-ioniques et leur utilisation.**

(30) Priorité : **26.01.89 FR 8900963**

(43) Date de publication de la demande : **01.08.90 Bulletin 90/31**

(45) Mention de la délivrance du brevet : **11.11.92 Bulletin 92/46**

(84) Etats contractants désignés : **AT BE CH DE DK ES FR GB IT LI NL SE**

(56) Documents cités :
**EP-A- 0 018 342**
**FR-A- 1 484 723**

(73) Titulaire : **L'OREAL**
**14, Rue Royale**
**F-75008 Paris (FR)**

(72) Inventeur : **Sebag, Henri**
**26, rue Erlanger**
**F-75016 Paris (FR)**

(74) Mandataire : **Casalonga, Axel et al**
**BUREAU D.A. CASALONGA - JOSSE**
**Morassistrasse 8**
**W-8000 München 5 (DE)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



Jouve, 18, rue Saint-Denis, 75001 PARIS

## Description

L'invention a pour objet un nouveau procédé de préparation de composés tensio-actifs non-ioniques, de nouveaux composés tensio-actifs non-ioniques, les compositions les contenant ainsi que l'utilisation de ces compositions dans l'industrie des cosmétiques et des produits pharmaceutiques.

Le nouveau procédé consiste dans une première étape, à condenser sur un composé comportant un groupement carboxy terminal, l'isopropylidène-1,2 époxypropyl-3 glycérol de formule (II) :

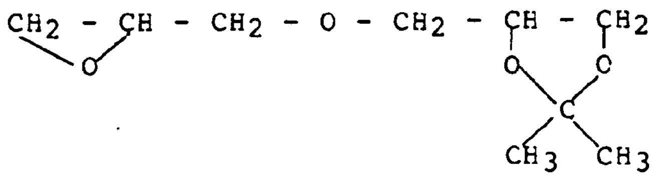

(II)

puis, dans une deuxième étape, à hydrolyser les produits obtenus, pour préparer des produits non-ioniques polyhydroxylés.

On connaît des composés tensio-actifs non-ioniques polyhydroxylés. En particulier, le brevet français n° 1 484 723 et le brevet US n° 3 674 902 décrivent des composés tensio-actifs de formule :

$$R - O - \left[ C_2H_3O(CH_2OCH_2CHOH-CH_2OH) \right]_n - H \qquad (F_1)$$

Ces produits de formule ($F_1$) sont obtenus par réaction de l'allylglycidyléther avec un alcool gras saturé ou insaturé, puis, par hydroxylation des doubles liaisons avec de l'eau oxygénée à 130 volumes, en présence d'acide formique à 98% pendant 24 à 48 heures à 40°C. L'utilisation d'eau oxygénée et d'acide concentrés présente toujours des risques tant du point de vue de la sécurité, que du point de vue de la formation des produits dus à une peroxydation ou dérivés de la réaction secondaire d'époxydation. De plus, au cours de cette 2ème étape, dans les cas où l'on utilise des alcools gras insaturés comme l'alcool oléique, par exemple, la double liaison est hydroxylée, conduisant à la formation de deux groupements hydroxyle au milieu de la chaîne grasse; on ne peut donc pas obtenir des composés de formule ($F_1$) dans laquelle R désigne un radical aliphatique insaturé.

Le procédé de la présente invention ne présente pas ces inconvénients.

Le nouveau procédé consiste, dans une première étape, à condenser sur des composés de formule ($I_1$) dont le groupement carboxy est terminal, l'isopropylidène-1,2 époxypropyl-3 glycérol de formule (II), puis, dans une deuxième étape, à hydrolyser les produits obtenus de formule ($III_1$), selon le schéma général suivant, en produits non-ioniques polyhydroxylés de formule ($IV_1$) :

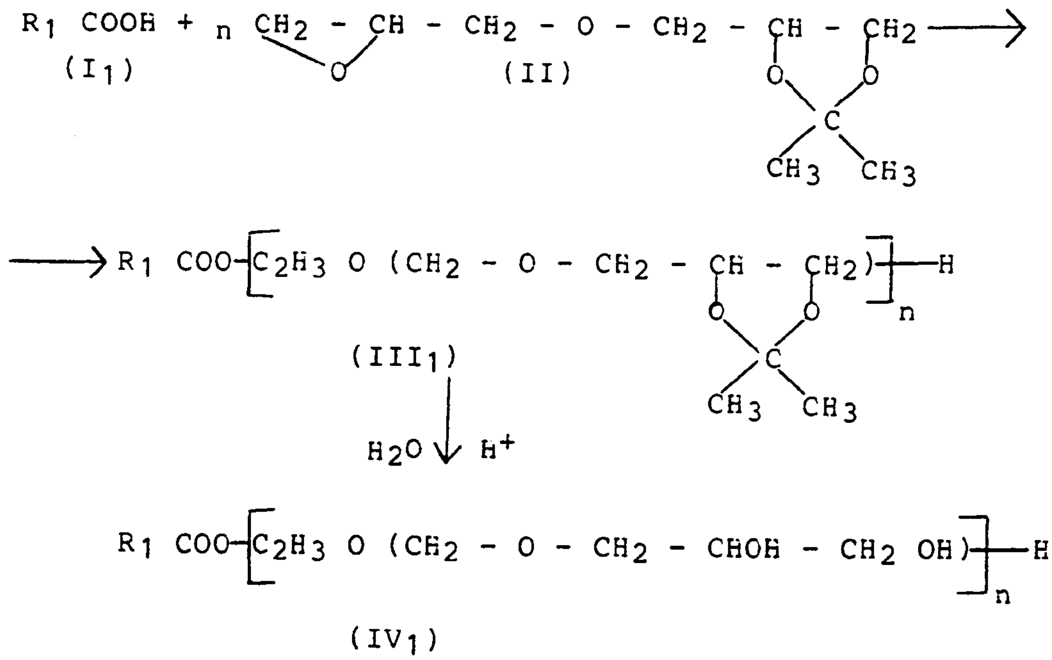

(I1) (II) (III1) (IV1)

Le groupement

$$C_2H_3O(CH_2\text{-}O\text{-}CH_2\text{-}CHOH\text{-}CH_2OH) \qquad (V)$$

désigne l'un ou l'autre des groupements suivants $V_a$ et $V_b$, résultant des deux sens d'ouverture possibles de la fonction époxyde de (II).

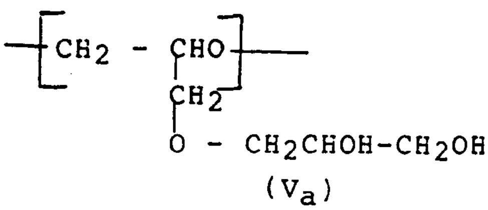
($V_a$)

et

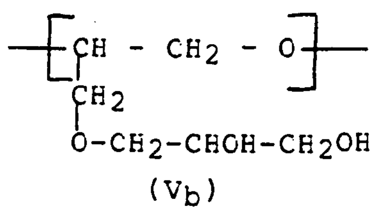
($V_b$)

n désigne un nombre entier ou décimal de 1 à 10 et de préférence de 1,5 à 8, et représente le nombre de moles d'époxyde de formule (II) mises en oeuvre par mole d'acide carboxylique de formule ($I_1$). Il représente dans les produits de formule ($IV_1$) le nombre moyen de motifs (V) par chaîne grasse, le nombre réel pouvant être inférieur, égal ou supérieur à ce dernier.

$R_1$ est un radical dérivé des acides carboxyliques de formule globale $R_1$-COOH ($I_1$) dont la formule générale développée peut être représentée par la formule (I) ci-après.

$$R-\left[Y(OH)-\right]_p\left[Z-\right]_q\left[X-CH_2\right]_v-COOH \qquad (I)$$

dans laquelle :

- R désigne un radical aliphatique, un radical cycloaliphatique ou alcoylarylique en $C_8$ à $C_{30}$;
- Y désigne un radical trivalent, tel que

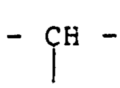 , 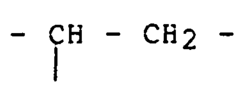 ou 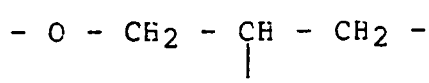

Dans chaque cas, le groupement hydroxyle est lié par liaison covalente au carbone tertiaire.

- Z désigne

$$-\left[OCH_2-CH_2\right]_m-$$

où m désigne un nombre entier ou décimal de 1 à 20.

- X désigne -O-, -S- ou S=O
- p, q et v désignent 0 ou 1, p+q= 0 ou 1

Quand Y désigne

$$-\underset{|}{CH}- \quad \text{ou} \quad -\underset{|}{CH}-CH_2-$$

alors

R désigne un radical aliphatique linénaire et saturé

Quand Y désigne

$$-\underset{|}{CH}-$$

et p=1, alors v=0.

Les composés tensio-actifs de l'invention peuvent donc être représentés par la formule générale (IV) suivante :

$$R-\left[Y\ (OB)\right]_p-\left[Z\right]_q-\left[X-CH_2\right]_v-COO\ A \qquad (IV)$$

dans laquelle R, X, Y, Z, p, q, v ont la même signification que ci-dessus,

A désigne

$$-\left[C_2H_3O\ (CH_2\ O\ CH_2-\ CHOH-CH_2\ OH)\right]_a-H$$

et B désigne

$$-\left[C_2H_3O\ (CH_2\ O\ CH_2-\ CHOH-CH_2\ OH)\right]_b-H$$

a + b = n.

En effet, quand p=1, c'est-à-dire quand l'acide carboxylique comporte un groupement hydroxyle, ce dernier est également susceptible de réagir avec l'époxyde de formule (II) et d'initier une autre chaîne hydrophile; dans ces cas, les composés de formule (IV) comportent deux chaînons hydrophiles par chaîne grasse hydrocarbonée.

Parmi les acides de formule (I) utilisables selon l'invention, on peut citer à titre d'exemple :

les acides alcanoïques, les acides alcénoïques, les acides alcoylpolyénoïques, les acides $\alpha$-hydroxyalcanoïques, les acides résiniques, les acides de lanoline;

les acides alcoylpolyoxyéthyloxy méthylène carboxyliques,

les acides alcoylphénylpolyoxyéthyloxy méthylène carboxyliques;

les acides alcoyloxy-3 hydroxy-2 propyloxy méthylène carboxyliques;
les acides alcoylphényloxy-3 hydroxy-2 propyloxyméthylène carboxyliques;
les acides alcoyloxy-3 hydroxy-2 propylthio méthylène carboxyliques;
les acides alcoylphényloxy-3 hydroxy-2 propylthiométhylène carboxyliques;
les acides hydroxy-2 alcoylthiométhylène carboxyliques.

Le procédé de l'invention permet d'obtenir, de façon simple, directement à partir d'acides carboxyliques de poids moléculaire éventuellement élevé, des tensio-actifs non-ioniques polyhydroxylés, dégradables, comportant essentiellement une chaîne grasse par bloc hydrophile.

Les produits de l'invention sont des tensio-actifs qui peuvent être solubles ou dispersibles dans l'eau selon la nature de la chaîne hydrocarbonée R et du nombre n de motifs hydrophiles.

Pour un acide $R_1$- COOH donné, plus n sera élevée, plus les produits seront hydrophiles et moins ils auront d'affinité pour les huiles, et inversement.

Le procédé de l'invention consiste à faire réagir par introduction lente l'époxyde de formule (II) avec un acide de formule (I).

La réaction se déroule sous atmosphère d'azote de préférence, en présence d'un catalyseur alcalin comme le méthylate, éthylate ou t-butylate de sodium ou potassium, à une température comprise entre 80° et 155°C.

La réaction peut être réalisée sans solvant ou bien dans un solvant apolaire comme le benzène, le toluène ou le xylène.

On utilise des quantités de 3 à 10% molaires de catalyseur par rapport à l'acide de formule (I).

La deuxième étape de la réaction consiste à hydrolyser les composés intermédiaires de formule ($III_1$). Après avoir éliminé le solvant éventuel, la masse réactionnelle est reprise dans un autre solvant, en présence de 2 à 50% d'eau et d'un acide minéral ou organique. On utilise de préférence comme solvant pour reprendre la masse réactionnelle, un alcool inférieur tel que l'isopropanol. Comme acide minéral on utilisera un acide, tel que l'acide chlorhydrique et comme acide organique un acide, tel que l'acide acétique, l'acide lactique, l'acide méthane sulfonique.

La réaction d'hydrolyse est faite à une température comprise entre 15 et 80°C et de préférence entre 20 et 50°C pendant 2 à 20 heures.

L'obtention de produits de formule (IV) dans laquelle X désigne S=O, est réalisée par oxydation des produits de formule (IV) où X représente un atome de soufre, avec de l'eau oxygénée à 35% et à une température de 20 à 50°C.

Les produits tensio-actifs sont ensuite obtenus par élimination des solvants et séchage sous pression réduite après neutralisation du catalyseur.

L'invention a également pour objet les produits non ioniques polyhydroxylés de formule (IV).

Ils sont solubles ou dispersibles dans l'eau. Selon les cas, les produits présentent des propriétés moussantes, émulsionnantes, dispersantes. Ils peuvent d'autre part être auto-émulsionnables, c'est-à-dire se disperser facilement dans l'eau en donnant des dispersions laiteuses stables se présentant éventuellement sous forme de microdispersions vésiculaires susceptibles de véhiculer des substances actives.

Les nouveaux produits non-ioniques de l'invention peuvent être utilisés comme tensio-actifs dans des compositions cosmétiques pour les soins de la peau et des cheveux telles que les compositions moussantes, comme les shampooings, les bains moussants, les compositions nettoyantes, des laits ou des crèmes pour le visage ou pour le corps, des compositions tinctoriales ou de coloration de la peau, des compositions solaires, des compositions adoucissantes ou démêlantes.

Ces composés tensio-actifs peuvent être aisément associés aux différents constituants généralement présents dans les compositions cosmétiques ou pharmaceutiques.

Ils peuvent être associés à d'autres tensio-actifs non-ioniques, à des tensio-actifs ioniques, à des polymères naturels ou synthétiques, à des huiles ou des cires minérales, animales ou végétales ou à des dérivés de polysiloxanes.

L'invention a également pour objet les compositions renfermant un ou plusieurs composés de formule (IV).

Ces compositions peuvent contenir en outre des solvants alcooliques, des propulseurs, des épaississants, des conservateurs, des filtres solaires, des colorants, des parfums, des additifs comme des émulsionnants, des stérols, des hydratants, des produits actifs pour le traitement des affections de la peau ou du cuir chevelu, pour la repousse des cheveux, etc...

Les compositions selon l'invention peuvent se présenter sous des formes diverses, notamment sous forme de solution, de lotion hydroalcoolique, d'émulsion huile-dans-eau ou eau-dans-huile, de microémulsion, de microdispersion de vésicules lipidiques renfermant facultativement des produits actifs éventuellement en présence d'huile, de gel, de pain ou de produits à pulvériser.

L'invention a également pour objet l'utilisation des compositions renfermant un composé de formule (IV) ou un produit résultant du procédé de préparation décrit ci-dessus, plus particulièrement dans les domaines

cosmétiques ou pharmaceutiques.

L'invention sera mieux comprise à l'aide des exemples non limitatifs ci-après.

## EXEMPLES DE PREPARATION

### EXEMPLE 1

Préparation d'un mélange de composés de formule (IV), dans laquelle :

R désigne le radical $C_{15}H_{31}$, p=q=v=o, a=n=3.

A 17,5 g d'acide palmitique (0,07 mole), on ajoute 1 g de méthylate de sodium en solution méthanolique (5,6 meq.), puis à 80°C, en 30 minutes, 13,16 g (0,07 mole) d'époxyde de formule (II). On élève progressivement la température à 145°-150°C, puis on ajoute encore 26,32 g (0,14 mole) d'époxyde de formule (II), en l'espace d'une heure. On maintient la température et l'agitation pendant 2 heures.

On reprend la masse réactionnelle avec 30 ml d'isopropanol et 30 ml d'acide chlorhydrique normal, en trois fractions, à 1 heure d'intervalle, et en chauffant légèrement à 30°-35°C.

On neutralise l'acidité avec de la soude, puis on évapore les solvants sous pression réduite.

On obtient une pâte de couleur beige, soluble dans l'eau avec très légère opalescence.

### EXEMPLE 2

Préparation d'un mélange de composés de formule (IV), dans laquelle :

R désigne le radical hydrocarboné dérivé de l'acide abiétique, p=q=v=o, a=n=4.

A 15,1 g d'acide abiétique (0,05 mole) fondu, on ajoute 0,71 g de liqueur méthanolique de méthylate de sodium à 5,6 meq./g, puis à la température de 125-130°C, 9,4 g (0,05 mole) d'époxyde de formule (II), en 30 minutes. On chauffe pendant une heure progressivement jusqu'à 150°C, puis on ajoute 28,2 g (0,15 mole) d'époxyde de formule (II).

Après 8 heures de chauffage, la masse réactionnelle de couleur brun foncé, ainsi obtenue, est lavée avec deux fois 100 ml d'eau à 30-35°C, en présence de 10 ml de dichlorométhane.

La phase organique est reprise avec 50 ml d'isopropanol, 20 ml d'eau et 2 ml d'acide chlorhydrique concentré, pendant 3 heures à 40°C.

On neutralise par addition de soude concentrée, puis on évapore les solvants sous pression réduite.

On obtient une pâte collante et filante, fortement colorée, soluble dans l'eau.

### EXEMPLE 3

Préparation d'un mélange de composés de formule (IV), dans laquelle :

R désigne le radical hydrocarboné dérivé de l'acide oléique,

p=0 q=1 v=1

Z désigne

$$-(OCH_2-CH_2)_m-$$

où m=5 (valeur moyenne)

X = -O- ; a=n=4.

A 30 g (0,05 mole) d'acide oleyloxypolyéthoxy méthylène carboxylique vendu sous le nom d'AKYPO RO 50 par la Société CHEMY, on ajoute 0,71 g de liqueur méthanolique de méthylate de sodium à 5,6 meq./g, puis à 125°C, en 30 minutes, 9,4 g (0,05 mole) d'epoxyde de formule (II). On chauffe ensuite progressivement à 150°C, puis on ajoute 28,2 g (0,15 mole) d'epoxyde de formule (II).

On chauffe à 150°C pendant 4 heures. On rajoute encore 0,05 g de t-butylate de potassium et on poursuit le chauffage pendant 2 heures.

La masse réactionnelle est reprise avec 50 ml d'isopropanol et 20 ml d'acide chlorhydrique normal, et on chauffe sous agitation, pendant 8 heures, entre 25 et 30°C.

Après neutralisation et évaporation des solvants sous pression réduite, on obtient un produit de couleur foncée soluble dans l'eau.

EXEMPLE 4

Préparation d'un mélange de composés de formule (IV), dans laquelle :
R désigne le radical $C_{15}H_{31}$ p=q=v=0 et a=n=1.
A 75 g d'acide palmitique (0,3 mole), on ajoute 4,3 g de solution méthanolique de méthylate de sodium à 5,6 meq./g et on chauffe progressivement jusqu'à 120-125°C sous balayage d'azote pour éliminer le méthanol. On ajoute ensuite à cette même température, en 1 heure, 56,4 g d'époxyde de formule (II). On maintient le chauffage et l'agitation pendant 1 heure 30 minutes. On refroidit et reprend la masse réactionnelle avec 200 ml d'isopropanol auxquels on ajoute 30 ml d'acide chlorhydrique normal et on agite à 35°C pendant 3 heures.
On neutralise exactement l'acide en excès avec de la soude et on évapore les solvants sous pression réduite. On obtient ainsi une cire blanche dispersible dans l'eau.

EXEMPLES D'APPLICATION

EXEMPLE A : shampooing

| | | |
|---|---|---|
| - Composés de l'exemple 2 | | 3,0 g MA |
| - Tensio-actif non-ionique de formule : $R-CHOH-CH_2-O(CH_2-CHOH-CH_2O)_n-H$ où R est un alkyle en $C_9$-$C_{12}$ n désigne une valeur statistique moyenne de 3,5 | | 10,0 g MA |
| - Chlorure de diméthyl distéaryl ammonium | | 1,0 g MA |
| - NaOH qs pH=7,0 | | |
| - Eau | qsp | 100,0 g |

EXEMPLE B : lait adoucissant pour le corps

| | | |
|---|---|---|
| - Composés de l'exemple 1 | | 7,0 g MA |
| - Composés de l'exemple 4 | | 3,0 g MA |
| - Huile de vaseline | | 40,0 g |
| - Eau | qsp | 100,0 g |

Les composés des exemples 1 et 4 sont solubilisés dans l'huile de vaseline en chauffant jusqu'à 80-90°C. On refroidit vers 60°C et on ajoute l'eau, elle-même chauffée à 60°C, tout en agitant vigoureusement. On obtient une émulsion fine et stable.
MA = matière active.

## Revendications

### Revendications pour les Etats contractants suivants : AT, BE, CH, DE, DK, FR, GB, IT, LI, NL, SE

1. Procédé de préparation de composés tensio-actifs non-ioniques, caractérisé par le fait que dans une première étape, on condense sur un composé de formule $R_1$-COOH ($I_1$) dont le groupement carboxy est terminal, l'isopropylidène-1,2 époxypropyl-3 glycérol de formule (II), puis, dans une deuxième étape, on hydrolyse les produits intermédiaires de formule ($III_1$), selon le schéma réactionnel suivant, en produits non-ioniques polyhydroxylés de formule ($IV_1$) :

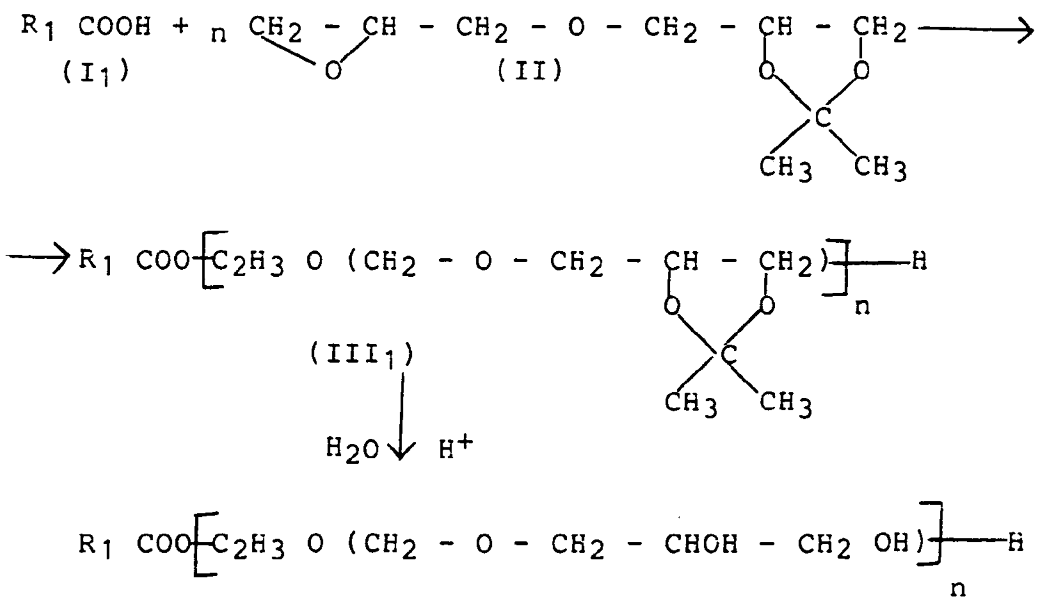


($I_1$) (II) ($III_1$) ($IV_1$)

le groupement

$$C_2H_3O(CH_2\text{-}O\text{-}CH_2\text{-}CHOH\text{-}CH_2OH) \qquad (V)$$

désigne les deux isomères :

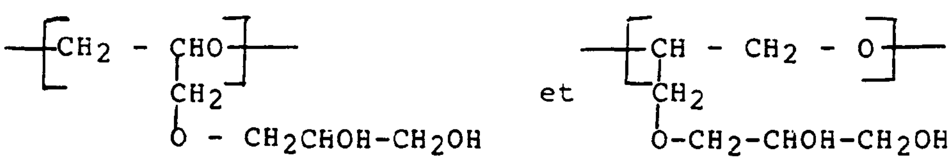

($V_a$) et ($V_b$)

n désigne un nombre entier ou décimal de 1 à 10;

$R_1$ est un radical dérivé des acides carboxyliques de formule globale $R_1$ COOH ($I_1$) dont la formule développée est :

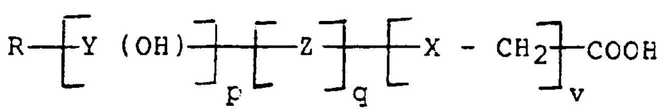

(I)

dans laquelle :

- R désigne un radical aliphatique, un radical cycloaliphatique ou alcoylarylique en $C_8$ à $C_{30}$;
- Y désigne un radical trivalent, tel que

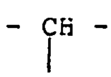 , 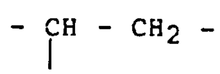 ou 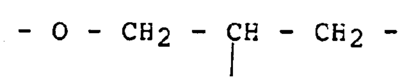

- Z désigne

$$-\left[OCH_2 - CH_2\right]_m-$$

où m désigne un nombre entier ou décimal de 1 à 20;
- X désigne -O-, -S- ou S=O;
- p, q et v désignent 0 ou 1 et p+q= 0 ou 1;
  quand Y désigne

$$-\underset{|}{C}H- \quad \text{ou} \quad -\underset{|}{C}H-CH_2,$$

R désigne un radical aliphatique linénaire et saturé, et
quand Y désigne

$$-\underset{|}{C}H-$$

et p=1, alors v=0

2. Procédé selon la revendication 1, caractérisé par le fait que la réaction d'addition de l'epoxyde de formule (II) sur l'acide carboxylique ($I_1$) est effectuée en présence d'un catalyseur alcalin, tel que le méthylate, l'éthylate ou le t-butylate de sodium ou de potassium, à une température comprise entre 80 et 155°C.

3. Procédé selon la revendication 1 ou 2, caractérisé par le fait que la réaction est effectuée sans solvant.

4. Procédé selon la revendication 1 ou 2, caractérisé par le fait que la réaction est effectuée en présence d'un solvant apolaire.

5. Procédé selon l'une quelconque des revendications 1 à 4, caractérisé par le fait au'on utilise des quantités de 3 à 10% molaires de catalyseur par rapport au réactif acide.

6. Procédé selon la revendication 1, caractérisé par le fait que l'hydrolyse des composés intermédiaires de formule ($III_1$) s'effectue en présence de 2 à 50% en poids d'eau et d'un acide minéral ou organique à une température comprise entre 15 et 80°C.

7. Procédé de préparation de produits tensio-actifs non-ioniques de formule générale :

$$R-\left[Y\ (OE)\right]_p-\left[Z\right]_q-\left[X - CH_2\right]_v-COO\ A \qquad (IV)$$

où :
- R désigne un radical aliphatique, un radical cycloaliphatique ou alcoylarylique en $C_8$ à $C_{30}$;
- Y désigne un radical trivalent, tel que

$$-\underset{|}{C}H-, \quad -\underset{|}{C}H-CH_2- \quad \text{ou} \quad -O-CH_2-\underset{|}{C}H-CH_2-;$$

- Z désigne

$$-\left[OCH_2 - CH_2\right]_m-$$

où m désigne un nombre entier ou décimal de 1 à 20;
- X désigne -O-, -S- ou S=O;

p, q et v désignent 0 ou 1; p+q = 0 ou 1;

Quand Y désigne

$$- \underset{|}{C}H - \text{ou} - \underset{|}{C}H - CH_2 -,$$

R désigne un radical aliphatique linéaire et saturé, et

quand Y désigne

$$- \underset{|}{C}H -$$

et p=1, alors v=0.

- A désigne

$$-\!\left[C_2H_3O\,(CH_2\,O\,CH_2 - CHOH - CH_2\,OH)\right]_a\!-H$$

et

- B désigne

$$-\!\left[C_2H_3O\,(CH_2\,O\,CH_2\,CHOH - CH_2\,OH)\right]_b\!-H$$

a+b = n;

n désigne un nombre entier ou décimal de 1 à 10,

**8.** Composition comprenant un ou plusieurs composés de formule (IV) selon la revendication 7, dans un véhicule approprié.

**9.** Composition cosmétique ou pharmaceutique, caractérisée par le fait qu'elle comprend un ou plusieurs composés de formule (IV) dans un véhicule approprié.

**10.** Composition selon la revendication 8 ou 9, caractérisée par le fait qu'elle renferme également un ou plusieurs produits choisis parmi les tensio-actifs non-ioniques, les tensio-actifs ioniques, les polymères naturels et synthétiques, les huiles et les cires minérales, animales ou végétales, les dérivés de polysiloxanes, les solvants, les propulseurs, les épaississants, les conservateurs, les filtres solaires, les colorants, les parfums, les agents émulsionnants, les stérols, les produits hydratants, les produits actifs pour le traitement des affections de la peau ou du cuir chevelu, les produits pour la repousse des cheveux.

**11.** Composition selon les revendications 9 ou 10, caractérisée par le fait qu'elle se présente sous forme de solution aqueuse, de lotion hydroalcoolique, d'émulsion huile-dans-eau ou eau-dans-huile, de microémulsion, de microdispersion de vésicules lipidiques contenant ou ne contenant pas de produits actifs, de gel, de pain ou de produits à pulvériser.

**Revendications pour l'Etat contractant suivant : ES**

**1.** Procédé de préparation de composés tensio-actifs non-ioniques, caractérisé par le fait que dans une première étape, on condense sur un composé de formule $R_1$-COOH ($I_1$) dont le groupement carboxy est terminal, l'isopropylidène-1,2 époxypropyl-3 glycérol de formule (II), puis, dans une deuxième étape, on hydrolyse les produits intermédiaires de formule ($III_1$), selon le schéma réactionnel suivant, en produits non-ioniques polyhydroxylés de formule ($IV_1$) :

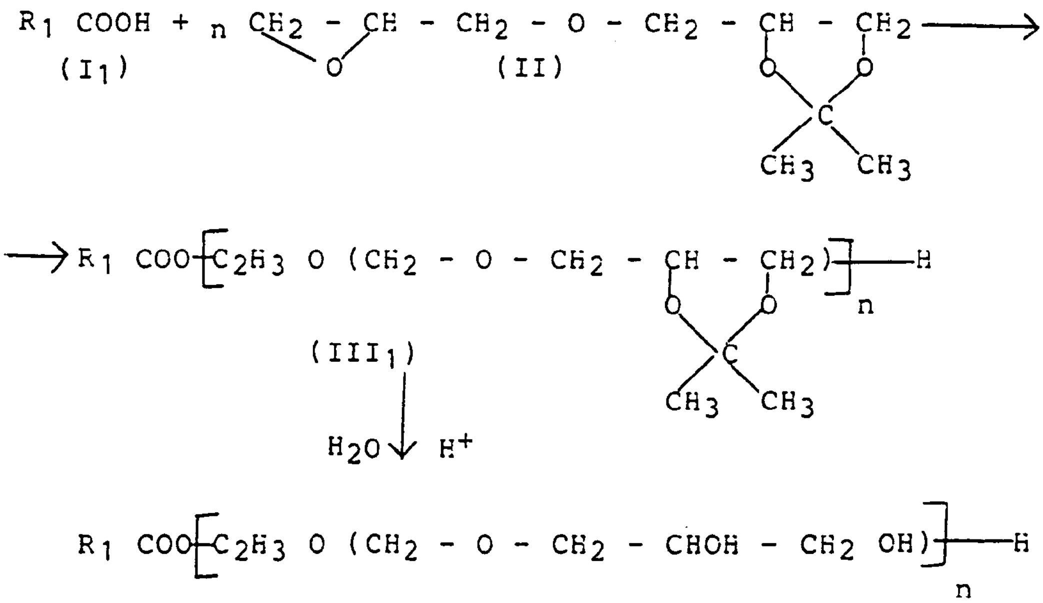


($I_1$) ($II$) ($III_1$)

($IV_1$)

le groupement

$$C_2H_3O(CH_2\text{-}O\text{-}CH_2\text{-}CHOH\text{-}CH_2OH) \qquad (V)$$

désigne les deux isomères :

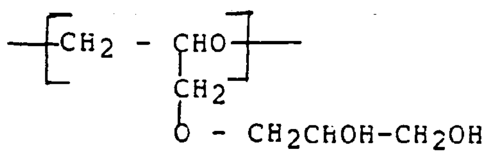 et 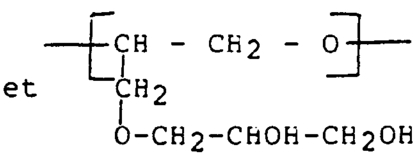

($V_a$) ($V_b$)

n désigne un nombre entier ou décimal de 1 à 10;

$R_1$ est un radical dérivé des acides carboxyliques de formule globale $R_1$ COOH ($I_1$) dont la formule développée est :

$$R-\left[Y(OH)\right]_p-\left[Z\right]_q-\left[X-CH_2\right]_v-COOH \qquad (I)$$

dans laquelle :

- R désigne un radical aliphatique, un radical cycloaliphatique ou alcoylarylique en $C_8$ à $C_{30}$;
- Y désigne un radical trivalent, tel que

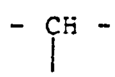 / 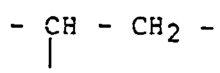 ou 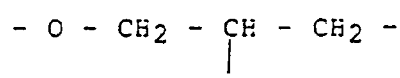

- Z désigne

$$-\left[OCH_2-CH_2\right]_m-$$

où m désigne un nombre entier ou décimal de 1 à 20;
- X désigne -O-, -S- ou S=O;
- p, q et v désignent 0 ou 1 et p+q= 0 ou 1;
quand Y désigne

$$-\underset{|}{C}H- \quad \text{ou} \quad -\underset{|}{C}H-CH_2,$$

R désigne un radical aliphatique linénaire et saturé, et
quand Y désigne

$$-\underset{|}{C}H$$

et p=1, alors v=0

2. Procédé selon la revendication 1, caractérisé par le fait que la réaction d'addition de l'epoxyde de formule (II) sur l'acide carboxylique ($I_1$) est effectuée en présence d'un catalyseur alcalin, tel que le méthylate, l'éthylate ou le t-butylate de sodium ou de potassium, à une température comprise entre 80 et 155°C.

3. Procédé selon la revendication 1 ou 2, caractérisé par le fait que la réaction est effectuée sans solvant.

4. Procédé selon la revendication 1 ou 2, caractérisé par le fait que la réaction est effectuée en présence d'un solvant apolaire.

5. Procédé selon l'une quelconque des revendications 1 à 4, caractérisé par le fait qu'on utilise des quantités de 3 à 10% molaires de catalyseur par rapport au réactif acide.

6. Procédé selon la revendication 1, caractérisé par le fait que l'hydrolyse des composés intermédiaires de formule ($III_1$) s'effectue en présence de 2 à 50% en poids d'eau et d'un acide minéral ou organique à une température comprise entre 15 et 80°C.

7. Procédé de préparation de produits tensio-actifs non-ioniques de formule générale :

$$R\!-\!\left[Y(OB)\right]_p\!-\!\left[Z\right]_q\!-\!\left[X-CH_2\right]_v\!-\!COO\ A \qquad (IV)$$

où :
- R désigne un radical aliphatique, un radical cycloaliphatique ou alcoylarylique en $C_8$ à $C_{30}$;
- Y désigne un radical trivalent, tel que

$$-\underset{|}{C}H- \ , \quad -\underset{|}{C}H-CH_2- \quad \text{ou} \quad -O-CH_2-\underset{|}{C}H-CH_2-;$$

- Z désigne

$$-\left[OCH_2-CH_2\right]_m-$$

où m désigne un nombre entier ou décimal de 1 à 20;
- X désigne -O-, -S- ou S=O;
p, q et v désignent 0 ou 1; p+q = 0 ou 1;
Quand Y désigne

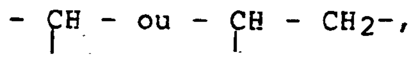

R désigne un radical aliphatique linéaire et saturé, et
quand Y désigne

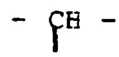

et p=1, alors v=0.

- A désigne

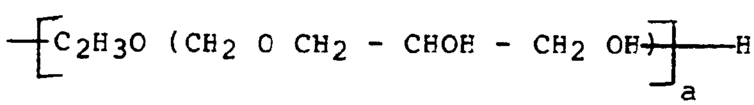

et

- B désigne

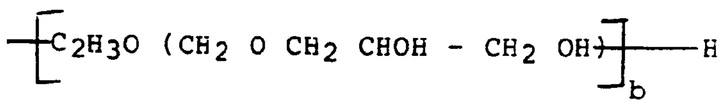

a+b = n; n désigne un nombre entier ou décimal de 1 à 10,
caractérisé par le fait que :

- dans une première étape, on condense sur une mole de composé de formule :

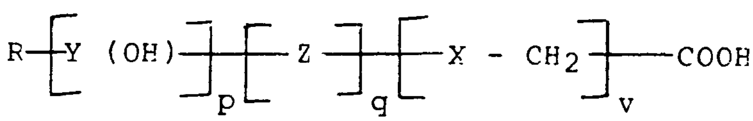

(I)

dans laquelle :

R, Y, Z, p, q, v ont les significations ci-dessus indiquées,
X désigne -O- ou -S-
n moles d'isopropylidène-1,2 époxypropyl-3 glycérol, de formule :

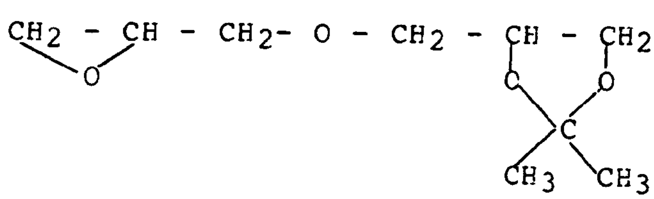

et

- dans une deuxième étape, on hydrolyse les produits résultants en présence d'un acide, pour obtenir les produits tensio-actifs non-ioniques de formule (IV).

8. Composition comprenant un ou plusieurs composés de formule (IV) préparés selon la revendication 7, dans un véhicule approprié.

9. Composition cosmétique ou pharmaceutique, caractérisée par le fait qu'elle comprend un ou plusieurs composés de formule (IV) dans un véhicule approprié.

**10.** Composition selon la revendication 8 ou 9, caractérisée par le fait qu'elle renferme également un ou plusieurs produits choisis parmi les tensio-actifs non-ioniques, les tensio-actifs ioniques, les polymères naturels et synthétiques, les huiles et les cires minérales, animales ou végétales, les dérivés de polysiloxanes, les solvants, les propulseurs, les épaississants, les conservateurs, les filtres solaires, les colorants, les parfums, les agents émulsionnants, les stérols, les produits hydratants, les produits actifs pour le traitement des affections de la peau ou du cuir chevelu, les produits pour la repousse des cheveux.

**11.** Composition selon les revendications 9 ou 10, caractérisée par le fait qu'elle se présente sous forme de solution aqueuse, de lotion hydroalcoolique, d'émulsion huile-dans-eau ou eau-dans-huile, de microémulsion, de microdispersion de vésicules lipidiques contenant ou ne contenant pas de produits actifs, de gel, de pain ou de produits à pulvériser.

## Patentansprüche

### Patentansprüche für folgende Vertragsstaaten : AT, BE, CH, DE, DK, FR, GB, IT, LI, NL, SE

**1.** Verfahren zur Herstellung von nicht-ionischen oberflächenaktiven Verbindungen, dadurch gekennzeichnet, daß in einem ersten Schritt eine Verbindung der Formel $R_1$-COOH ($I_1$), deren Carboxygruppierung endständig ist, mit Isopropyliden-1,2-Epoxypropyl-3-glycerin der Formel (II) kondensiert wird, anschließend in einem zweiten Schritt die Zwischenprodukte der Formel ($III_1$) gemäß dem folgenden Reaktionsschema zu nicht-ionischen polyhydroxylierten Produkten der Formel ($IV_1$) hydrolisiert werden:

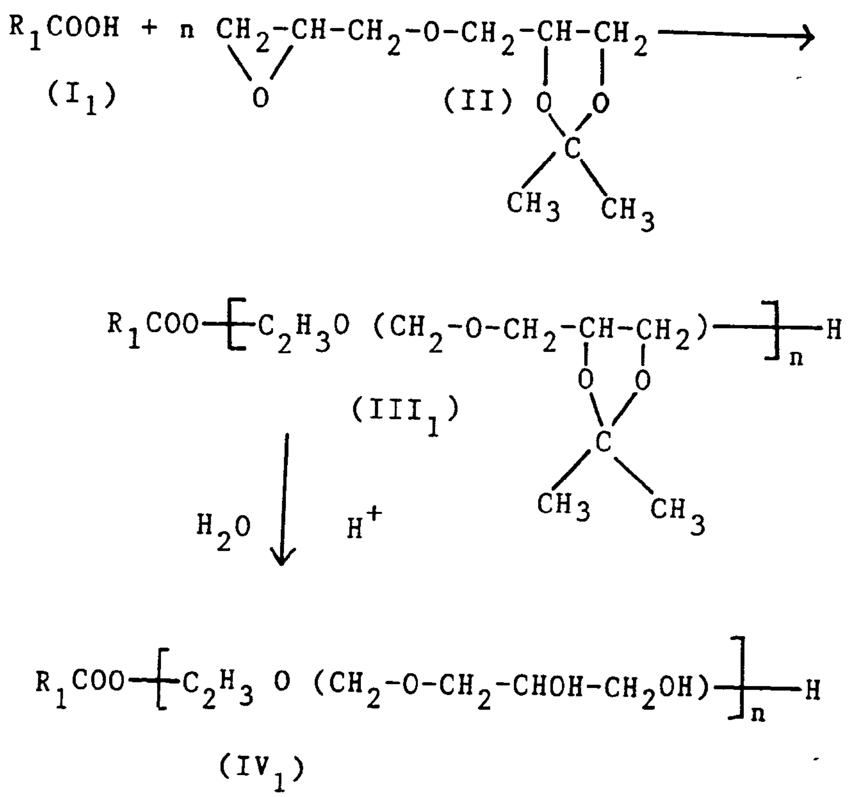


($I_1$) (II)

($III_1$)

($IV_1$)

die Gruppierung

$$C_2H_3O(CH_2\text{-}O\text{-}CH_2\text{-}CHOH\text{-}CH_2OH) \quad (V)$$

bezeichnet die beiden Isomeren:

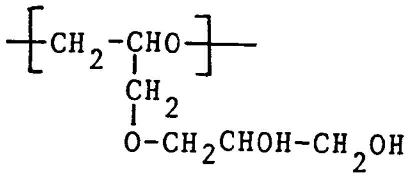

$(V_a)$

und

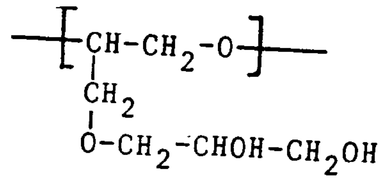

$(V_b)$

n bezeichnet eine ganze oder Dezimal-Zahl von 1 bis 10;
$R_1$ ist eine radikalische Gruppe, die von Carbonsäuren der Gesamt-Formel $R_1COOH$ ($I_1$) abgeleitet ist, und deren Formel wie folgt erläutert ist:

$$R-\left[Y(OH)\right]_p-\left[Z\right]_q-\left[X-CH_2\right]_v-COOH \quad (I)$$

in der

- R eine radikalische aliphatische, eine radikalische cycloaliphatische oder Alkoyl-aryl-Gruppe aus $C_8$ bis $C_{30}$ bezeichnet;
- Y eine trivalente radikalische Gruppe wie

$$-\underset{|}{C}H- \quad -\underset{|}{C}H-CH_2- \text{ oder } -O-CH_2-\underset{|}{C}H-CH_2-$$

bezeichnet;
- Z

$$\left[-OCH_2-CH_2-\right]_m$$

bezeichnet, wobei m eine ganze oder Dezimal-Zahl von 1 bis 20 bedeutet;
- X -0-, -S- oder S = 0 bezeichnet;
- p, q und v 0 oder 1 bezeichnen und p + q = 0 oder 1;

wenn Y

$$-\underset{|}{C}H- \text{ oder } -\underset{|}{C}H-CH_2$$

bezeichnet,
R eine radikalische, aliphatische lineare und gesättigte Gruppe bezeichnet, und wenn Y

$$-\underset{|}{C}H-$$

bezeichnet und p gleich 1 ist, dann ist v gleich 0.

2. Verfahren nach Anspruch 1, dadurch gekennzeichnet, daß die Reaktion der Addition von Epoxid der Formel (II) an die Carbonsäure ($I_1$) in Gegenwart eines alkalischen Katalysators wie z.B. Natrium- oder Kalium-methylat, -ethylat oder -t-butylat, bei einer Temperatur im Bereich von 80 bis 155°C durchgeführt wird.

3. Verfahren nach Anspruch 1 oder 2, dadurch gekennzeichnet, daß die Reaktion ohne Lösungsmittel durchgeführt wird.

4. Verfahren nach Anspruch 1 oder 2, dadurch gekennzeichnet, daß die Reaktion in Gegenwart eines apolaren Lösungsmittels durchgeführt wird.

5. Verfahren nach einem der Ansprüche 1-4, dadurch gekennzeichnet, daß 3- bis 10- molare Mengen an

Katalysator, bezogen auf die reaktive Säure, verwendet werden.

6. Verfahren nach Anspruch 1, dadurch gekennzeichnet, daß die Hydrolyse der Zwischenprodukte der Formel ($III_1$) in Gegenwart von 2-50 Gew.% Wasser oder einer Mineral- oder organischen Säure bei einer Temperatur im Bereich von 15 bis 80°C durchgeführt wird.

7. Nicht-ionische oberflächenaktive Produkte der allgemeinen Formel

$$R-\left[Y\,(OB)\right]_p-\left[Z\right]_q-\left[X-CH_2\right]_v-COO\,A \qquad (IV)$$

worin

- R eine radikalische aliphatische, eine radikalische cycloaliphatische oder Alkoylaryl-Gruppe aus $C_8$ bis $C_{30}$ bezeichnet;
- Y eine radikalische trivalente Gruppe wie z.B.

$$-\underset{|}{C}H- \;,\; -\underset{|}{C}H-CH_2- \quad \text{oder} \quad -O-CH_2-\underset{|}{C}H-CH_2-$$

bezeichnet;
- Z

$$-\left[OCH_2-CH_2\right]_m-$$

bezeichnet, wobei m eine ganze oder Dezimal-Zahl von 1 bis 20 bezeichnet;
- X -O-, -S- oder S=0 bezeichnet;

p, q und v gleich 0 oder 1 sind; p + q = 0 oder 1; wenn Y

$$-\underset{|}{C}H- \quad \text{oder} \quad -\underset{|}{C}H-CH_2-$$

bezeichnet, R eine radikalische, aliphatische lineare und gesättigte Gruppe bezeichnet, und wenn Y

$$-\underset{|}{C}H-$$

und p gleich 1 ist, dann v gleich 0 ist;
- A

$$-\left[C_2H_3O(CH_2-O-CH_2-CHOH-CH_2OH)\right]_a H$$

und
- B

$$-\left[C_2H_3O(CH_2OCH_2CHOH-CH_2OH)\right]_b H$$

bezeichnet, wobei a + b gleich n ist,
n eine ganze oder Dezimal-Zahl von 1 bis 10 bezeichnet.

8. Zusammensetzung, die eine oder mehrere Verbindungen der Formel (IV) nach Anspruch 7 in einem geeigneten Vehikel enthält.

9. Kosmetische oder pharmazeutische Zusammensetzung, dadurch gekennzeichnet, daß sie eine oder mehrere Verbindungen der Formel (IV) in einem geeigneten Vehikel enthält.

10. Zusammensetzung nach Anspruch 8 oder 9, dadurch gekennzeichnet, daß sie auch ein oder mehrere Produkte enthält, die unter den nicht-ionischen oberflächenaktiven Mitteln, den ionischen oberflächenaktiven Mitteln, den natürlichen und synthetischen Polymeren, den Ölen und den mineralischen, tierischen oder pflanzlichen Wachsen, den Polysiloxanderivaten, den Lösungsmitteln, den Konservierungsmitteln, den Sonnenfiltern, den Farbstoffen, den Parfums, den emulgierenden Agenzien, den Sterinen, den

feuchtigkeitsspendenden Produkten, den Produkten, die zur Behandlung von Erkrankungen der Haut oder der Kopfhaut wirksam sind, den Produkten für den Haarwuchs ausgewählt sind.

11. Zusammensetzung nach den Ansprüchen 9 oder 10, dadurch gekennzeichnet, daß sie in Form einer wässrigen Lösung, einer hydroalkoholischen Emulsion, einer Öl-in-Wasser-Emulsion oder einer Wasser-in-Öl-Emulsion, einer Mikroemulsion von lipidischen Tröpfchen, die aktive Produkte enthalten oder nicht enthalten, in Form von Gel, in Form einer Masse oder in Form von zu pulverisierenden Produkten vorliegt.

**Patentansprüche für folgenden Vertragsstat : ES**

1. Verfahren zur Herstellung von nicht-ionischen oberflächenaktiven Verbindungen, dadurch gekennzeichnet, daß in einem ersten Schritt eine Verbindung der Formel $R_1$-COOH ($I_1$), deren Carboxygruppierung endständig ist, mit Isopropyliden-1,2-Epoxypropyl-3-glycerin der Formel (II) kondensiert wird, anschließend in einem zweiten Schritt die Zwischenprodukte der Formel ($III_1$) gemäß dem folgenden Reaktionsschema zu nicht-ionischen polyhydroxylierten Produkten der Formel ($IV_1$) hydrolisiert werden:

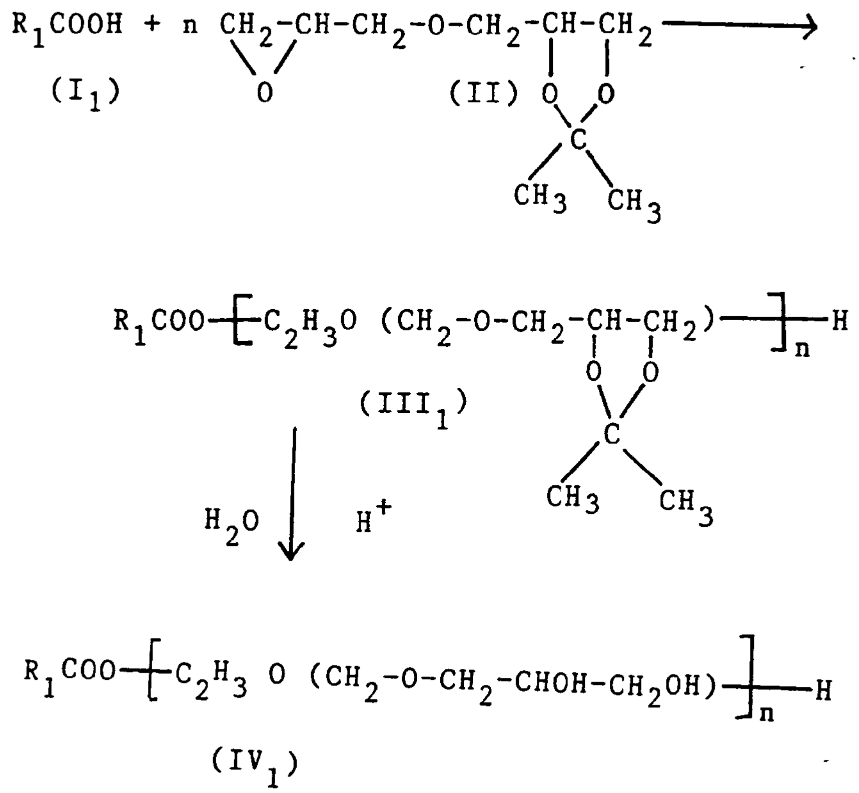

($I_1$) (II) ($III_1$) ($IV_1$)

die Gruppierung

$C_2H_3O(CH_2\text{-}O\text{-}CH_2\text{-}CHOH\text{-}CH_2OH)$ (V)

bezeichnet die beiden Isomeren:

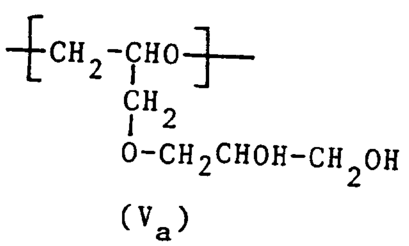
($V_a$)

und

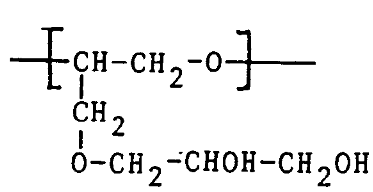
($V_b$)

n bezeichnet eine ganze oder eine Dezimal-Zahl von 1 bis 10;
$R_1$ ist eine radikalische Gruppe, die von Carbonsäuren der Gesamt-Formel $R_1COOH$ ($I_1$) abgeleitet ist,

und deren Formel wie folgt ist:

$$R-\left[Y(OH)\right]_p-\left[Z\right]_q-\left[X-CH_2\right]_v-COOH \quad (I)$$

in der

- R eine radikalische aliphatische, eine radikalische cycloaliphatische oder Alkoylaryl-Gruppe aus $C_8$ bis $C_{30}$ bezeichnet;
- Y eine trivalente radikalische Gruppe wie

$$-\underset{|}{C}H- \;,\; -\underset{|}{C}H-CH_2- \text{ oder } -O-CH_2-\underset{|}{C}H-CH_2-$$

bezeichnet;
- Z

$$-\!\left[OCH_2-CH_2\right]_m-$$

bezeichnet, wobei m eine ganze oder Dezimal-Zahl von 1 bis 20 bedeutet;
- X -0-, -S- oder S = 0 bezeichnet;
- p, q und v 0 oder 1 bezeichnen und p + q = 0 oder 1;

wenn Y

$$-\underset{|}{C}H- \text{ oder } -\underset{|}{C}H-CH_2$$

bezeichnet,

R eine radikalische, aliphatische lineare und gesättigte Gruppe bezeichnet, und wenn Y

$$-\underset{|}{C}H-$$

bezeichnet und p gleich 1 ist, dann v gleich 0 ist.

**2.** Verfahren nach Anspruch 1, dadurch gekennzeichnet, daß die Reaktion der Addition von Epoxid der Formel (II) an die Carbonsäure ($I_1$) in Gegenwart eines alkalischen Katalysators wie z.B. Natrium- oder Kalium-methylat, -ethylat oder -t-butylat, bei einer Temperatur im Bereich von 80 bis 155°C durchgeführt wird.

**3.** Verfahren nach Anspruch 1 oder 2, dadurch gekennzeichnet, daß die Reaktion ohne Lösungsmittel durchgeführt wird.

**4.** Verfahren nach Anspruch 1 oder 2, dadurch gekennzeichnet, daß die Reaktion in Gegenwart eines apolaren Lösungsmittels durchgeführt wird.

**5.** Verfahren nach einem der Ansprüche 1-4, dadurch gekennzeichnet, daß 3- bis 10- molare Mengen an Katalysator, bezogen auf die reaktive Säure, verwendet werden.

**6.** Verfahren nach Anspruch 1, dadurch gekennzeichnet, daß die Hydrolyse der Zwischenprodukte der Formel ($III_1$) in Gegenwart von 2-50 Gew.% Wasser und einer Mineral- oder organischen Säure bei einer Temperatur im Bereich von 15 bis 80°C durchgeführt wird.

**7.** Verfahren zur Herstellung von nicht-ionischen oberflächenaktive Produkte der allgemeinen Formel

$$R-\left[Y(OB)\right]_p-\left[Z\right]_q-\left[X-CH_2\right]_v-COO\,A \quad (IV)$$

worin

- R eine radikalische aliphatische, eine radikalische cycloaliphatische oder Alkoylaryl-Gruppe aus $C_8$ bis $C_{30}$ bezeichnet;
- Y eine radikalische trivalente Gruppe wie z.B.

$$-\overset{}{\underset{|}{C}}H- \ , \ -\underset{|}{C}H-CH_2- \quad \text{oder} \quad -O-CH_2-\underset{|}{C}H-CH_2-$$

bezeichnet;

- Z

$$-\!\!\left( OCH_2-CH_2 \right)\!\!-_m$$

bezeichnet, wobei m eine ganze oder Dezimal-Zahl von 1 bis 20 bezeichnet;

- X -O-, -S- oder S=O bezeichnet;

p, q und v gleich 0 oder 1 sind; p + q = 0 oder 1;

wenn Y

$$-\underset{|}{C}H- \quad \text{oder} \quad -\underset{|}{C}H-CH_2-$$

bezeichnet, R eine radikalische, aliphatische lineare und gesättigte Gruppe bezeichnet, und wenn Y -CH- und p gleich 1 ist, dann v gleich 0 ist;

- A

$$-\!\!\left( C_2H_3O(CH_2-O-CH_2-CHOH-CH_2OH) \right)\!\!-_a H$$

und

- B

$$-\!\!\left( C_2H_3O(CH_2OCH_2CHOH-CH_2OH) \right)\!\!-_b H$$

bezeichnet,

wobei a + b gleich n ist,

n eine ganze oder Dezimal-Zahl von 1 bis 10 bezeichnet,

dadurch gekennzeichnet, daß

- in einem ersten Schritt ein Mol der Verbindung der Formel

$$R-\left[ Y\,(OH) \right]_p-\left[ Z \right]_q-\left[ X - CH_2 \right]_v-COOH \qquad (I)$$

in der

R, Y, Z, p, q, v die oben angegebenen Bedeutungen haben,

X -0- oder -S- bezeichnet;

mit n Mol Isopropyliden-1,2-epoxipropyl-3-glycerin der Formel

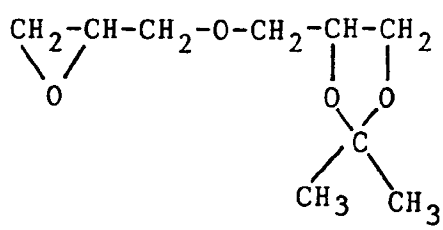

kondensiert werden und

- in einem zweiten Schritt die erhaltenen Produkte in Gegenwart einer Säure hydrolysiert, um die nichtionischen oberflächenaktiven Produkte der Formel (IV) zu erhalten.

**8.** Zusammensetzung, die eine oder mehrere Verbindungen der Formel (IV), die nach Anspruch 7 hergestellt wurden, in einem geeigneten Vehikel enthält.

**9.** Kosmetische oder pharmazeutische Zusammensetzung, dadurch gekennzeichnet, daß sie eine oder mehrere Verbindungen der Formel (IV) in einem geeigneten Vehikel enthält.

**10.** Zusammensetzung nach Anspruch 8 oder 9, dadurch gekennzeichnet, daß sie auch ein oder mehrere Produkte enthält, die unter den nicht-ionischen oberflächenaktiven Mitteln, den ionischen oberflächenaktiven Mitteln, den natürlichen und synthetischen Polymeren, den Ölen und den mineralischen, tierischen oder pflanzlichen Wachsen, den Polysiloxanderivaten, den Lösungsmitteln, den Treibmitteln, den Verdickungsmitteln, Konservierungsmitteln, den Sonnenfiltern, den Farbstoffen, den Parfums, den emulgierenden Agenzien, den Sterinen, den feuchtigkeitsspendenden Produkten, den Produkten, die zur Behandlung von Erkrankungen der Haut oder der Kopfhaut wirksam sind, den Produkten für den Haarwuchs ausgewählt sind.

**11.** Zusammensetzung nach den Ansprüchen 9 oder 10, dadurch gekennzeichnet, daß sie in Form einer wässrigen Lösung, einer hydroalkoholischen Emulsion, einer Öl-in-Wasser-Emulsion oder einer Wasser-in-Öl-Emulsion, einer Mikroemulsion von lipidischen Tröpfchen, die aktive Produkte enthalten oder nicht enthalten, in Form von Gel, in Form einer Masse oder in Form von zu pulverisierenden Produkten vorliegt.

## Claims

**Claims for the following Contracting States : AT, BE, CH, DE, DK, FR, GB, IT, LI, NL, SE**

**1.** Process for the preparation of nonionic surface-active compounds, characterised in that, in a first stage, 1,2-isopropylidene-3-epoxypropylglycerol of formula (II) is condensed with a compound of formula $R_1$-COOH ($I_1$) in which the carboxyl group is terminal, and then, in a second stage, the intermediate products of formula ($III_1$) are hydrolysed according to the following reaction scheme, to polyhydroxylated nonionic products of formula ($IV_1$):

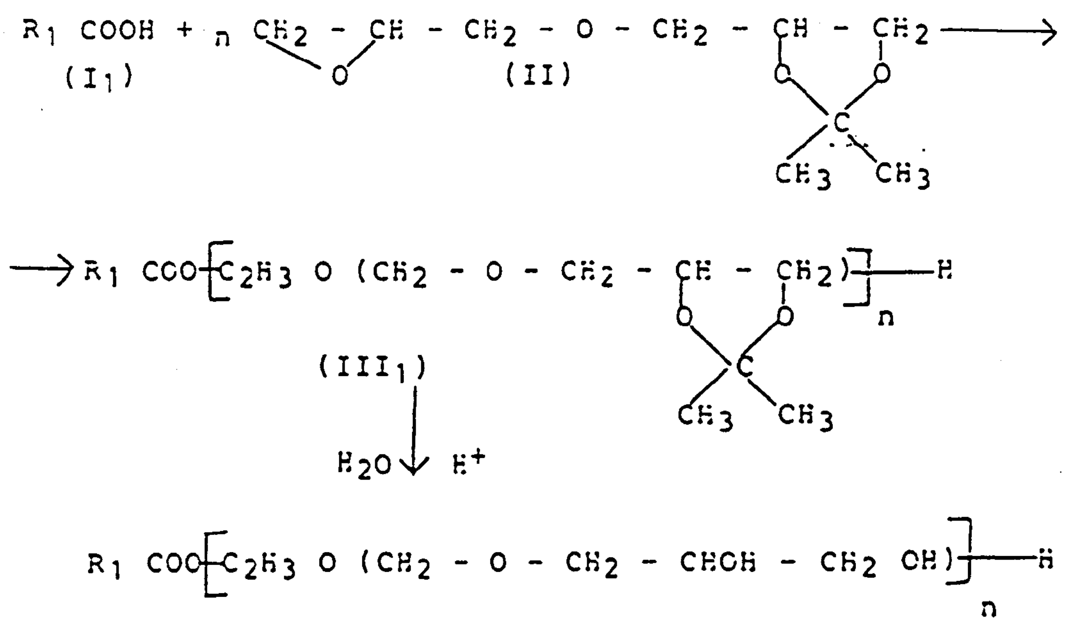


($I_1$) (II) ($III_1$) ($IV_1$)

the group

$$C_2H_3O(CH_2\text{-}O\text{-}CH_2\text{-}CHOH\text{-}CH_2OH) \quad (V)$$

denotes the two isomers:

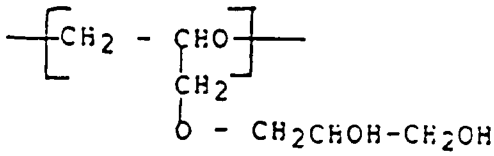

(Va)

and

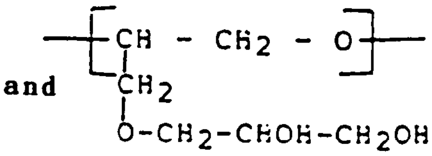

(Vb)

n denotes a whole or decimal number from 1 to 10;

$R_1$ is a radical derived from carboxylic acids of overall formula $R_1COOH$ ($I_1$) whose structural formula is:

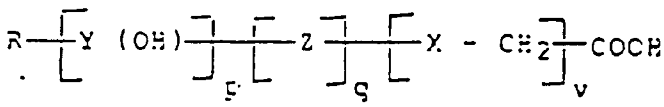

(I)

in which:

- R denotes an aliphatic radical or a cycloaliphatic or alkylaryl radical from $C_8$ to $C_{30}$;
- Y denotes a trivalent radical such as

$$-\underset{|}{C}H-,\quad -\underset{|}{C}H-CH_2-\ \text{or}\ -O-CH_2-\underset{|}{C}H-CH_2-$$

- Z denotes

$$-\!\!(OCH_2-CH_2)\!\!-_m$$

where m denotes a whole or decimal number from 1 to 20;
- X denotes -O-, -S- or S=O;
- p, q and v denotes 0 or 1 and p+q = 0 or 1;

when Y denotes

$$-\underset{|}{C}H-\ \text{or}\ -\underset{|}{C}H-CH_2,$$

R denotes a linear and saturated aliphatic radical, and

when Y denotes

$$-\underset{|}{C}H-$$

and p=1, then v=0.

2. Process according to Claim 1, characterised in that the reaction of addition of the epoxide of formula (II) to the carboxylic acid ($I_1$) is performed in the presence of an alkaline catalyst such as sodium or potassium methylate, ethylate or t-butylate, at a temperature of between 80 and 155°C.

3. Process according to Claim 1 or 2, characterised in that the reaction is performed without solvent.

4. Process according to Claim 1 or 2, characterised in that the reaction is performed in the presence of an apolar solvent.

5. Process according to any one of Claims 1 to 4, characterised in that quantities of 3 to 10 mol% of catalyst are employed, relative to the acidic reactant.

6. Process according to Claim 1, characterised in that the hydrolysis of the intermediate compounds of formula ($III_1$) is performed in the presence of 2 to 50 % weight of water and of an inorganic or organic acid

at a temperature of between 15 and 80°C.

7. Process for the preparation of nonionic surface-active products of general formula:

$$R-\left[Y(OB)\right]_p-\left[Z\right]_q-\left[X-CH_2\right]_v-COO\,A \quad (IV)$$

where:
- R denotes an aliphatic radical or a cycloaliphatic or alkylaryl radical from $C_8$ to $C_{30}$;
- Y denotes a trivalent radical such as

$$-\underset{|}{CH}-,\quad -\underset{|}{CH}-CH_2-\ \text{or}\ -O-CH_2-\underset{|}{CH}-CH_2-;$$

- Z denotes

$$-\!\left[OCH_2-CH_2\right]_m-$$

where m denotes a whole or decimal number from 1 to 20;
- X denotes -O-,-S- or S=O;
  p, q and v denote 0 or 1; p+q = 0 or 1;
  when Y denotes

$$-\underset{|}{CH}-\ \text{or}\ -\underset{|}{CH}-CH_2-,$$

  R denotes a linear and saturated aliphatic radical, and when Y denotes

$$-\underset{|}{CH}-$$

and p=1, then v=0.
- A denotes

$$-\!\left[C_2H_3O(CH_2OCH_2-CHOH-CH_2OH)\right]_a-H$$

and
- B denotes

$$-\!\left[C_2H_3O(CH_2OCH_2CHOH-CH_2OH)\right]_b-H$$

a+b = n,
n denotes a whole or decimal number from 1 to 10.

8. Composition comprising one or more compounds of formula (IV) according to Claim 7, in a suitable carrier.

9. Cosmetic or pharmaceutical composition characterised in that it comprises one or more compounds of formula (IV) in a suitable carrier.

10. Composition according to Claim 8 or 9, characterised in that it also includes one or more products chosen from nonionic surfactants, ionic surfactants, natural and synthetic polymers, mineral, animal or vegetable oils and waxes, polysiloxane derivatives, solvents, propellants, thickeners, preservatives, sunscreens, dyes, perfumes, emulsifying agents, sterols, hydrating products and products for the regrowth of hair.

11. Composition according to Claims 9 or 10, characterised in that it is in the form of aqueous solution, of hydroalcoholic lotion, of oil-in-water or water-in-oil emulsion, of microemulsion, of microdispersion of lipid vesicles containing or not containing active products, of gel, of cake or of products for spraying.

**Claims for the following Contracting State : ES**

1. Process for the preparation of nonionic surface-active compounds, characterised in that, in a first stage, 1,2-isopropylidene-3-epoxypropylglycerol of formula (II) is condensed with a compound of formula $R_1$-COOH ($I_1$) in which the carboxyl group is terminal, and then, in a second stage, the intermediate products of formula ($III_1$) are hydrolysed according to the following reaction scheme, to polyhydroxylated nonionic products of formula ($IV_1$):

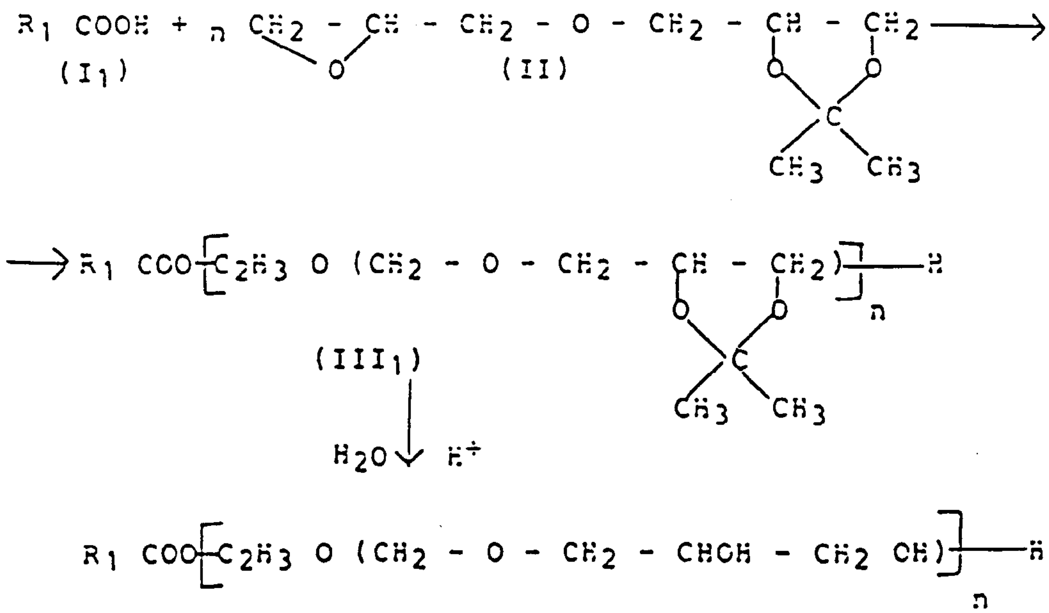


($IV_1$)

the group

$$C_2H_3O(CH_2\text{-}O\text{-}CH_2\text{-}CHOH\text{-}CH_2OH) \quad (V)$$

denotes the two isomers:

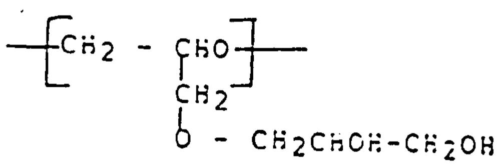
($V_a$)

and

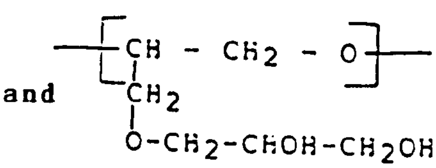
($V_b$)

n denotes a whole or decimal number from 1 to 10;
$R_1$ is a radical derived from carboxylic acids of overall formula $R_1COOH$ ($I_1$) whose structural formula is:

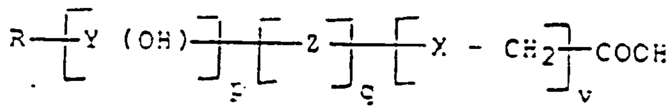
(I)

in which:
- R denotes an aliphatic radical or a cycloaliphatic or alkylaryl radical from $C_8$ to $C_{30}$;
- Y denotes a trivalent radical such as

$$-\underset{|}{C}H-,\ -\underset{|}{C}H-CH_2-\ \text{or}\ -O-CH_2-\underset{|}{C}H-CH_2-$$

- Z denotes

$$-\!\!\left[OCH_2-CH_2\right]\!\!-_m$$

where m denotes a whole or decimal number from 1 to 20;
- X denotes -O-, -S- or S=O;
- p, q and v denote 0 or 1 and p+q = 0 or 1;
  when Y denotes

$$-\underset{|}{CH}- \text{ or } -\underset{|}{CH}-CH_2,$$

R denotes a linear and saturated aliphatic radical, and
  when Y denotes

$$-\underset{|}{CH}-$$

and p=1, then v=0.

2. Process according to Claim 1, characterised in that the reaction of addition of the epoxide of formula (II) to the carboxylic acid ($I_1$) is performed in the presence of an alkaline catalyst such as sodium or potassium methylate, ethylate or t-butylate, at a temperature of between 80 and 155°C.

3. Process according to Claim 1 or 2, characterised in that the reaction is performed without solvent.

4. Process according to Claim 1 or 2, characterised in that the reaction is performed in the presence of an apolar solvent.

5. Process according to any one of Claims 1 to 4, characterised in that quantities of 3 to 10 mol% of catalyst are employed, relative to the acidic reactant.

6. Process according to Claim 1, characterised in that the hydrolysis of the intermediate compounds of formula ($III_1$) is performed in the presence of 2 to 50 % weight of water and of an inorganic or organic acid at a temperature of between 15 and 80°C.

7. Process for the preparation of nonionic surface-active products of general formula:

$$R-\left[Y\ (OE)\right]_p-\left[Z\right]_q-\left[X-CH_2\right]_v-COO\ A \qquad \text{(IV)}$$

where:
- R denotes an aliphatic radical or a cycloaliphatic or alkylaryl radical from $C_8$ to $C_{30}$;
- Y denotes a trivalent radical such as

$$-\underset{|}{CH}-,\ -\underset{|}{CH}-CH_2- \text{ or } -O-CH_2-\underset{|}{CH}-CH_2-;$$

- Z denotes

$$-\!\!\left[OCH_2-CH_2\right]\!\!-_m$$

where m denotes a whole or decimal number from 1 to 20;
- X denotes -O-,-S- or S=O;
p, q and v denote 0 or 1; p+q = 0 or 1;
  when Y denotes

$$-\underset{|}{CH}- \text{ or } -\underset{|}{CH}-CH_2-,$$

R denotes a linear and saturated aliphatic radical, and

when Y denotes

$$-\underset{|}{C}H-$$

and p=1, then v=0.

- A denotes

$$-[C_2H_3O(CH_2OCH_2-CHOH-CH_2OH)]_a-H$$

and

- B denotes

$$-[C_2H_3O(CH_2OCH_2CHOH-CH_2OH)]_b-H$$

a+b = n; n denotes a whole or decimal number from 1 to 10,
characterised in that:

- in a first stage, n moles of 1,2-isopropylidene-3-epoxypropylglycerol, of formula:

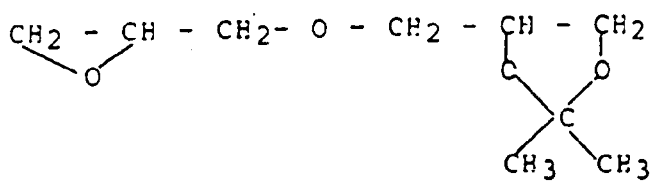

are condensed with one mole of compound of formula:

$$R-[Y(OH)]_p-[Z]_q-[X-CH_2]_v-COOH \qquad (I)$$

in which :

R, Y, Z, p, q and v have the meanings shown above,
X denotes -O- or -S-

and

- in a second stage, the resulting products are hydrolysed in the presence of an acid to obtain the nonionic surface-active products of formula (IV).

**8.** Composition comprising one or more compounds of formula (IV) prepared according to Claim 7, in a suitable carrier.

**9.** Cosmetic or pharmaceutical composition characterised in that it comprises one or more compounds of formula (IV) in a suitable carrier.

**10.** Composition according to Claim 8 or 9, characterised in that it also includes one or more products chosen from nonionic surfactants, ionic surfactants, natural and synthetic polymers, mineral, animal or vegetable oils and waxes, polysiloxane derivatives, solvents, propellants, thickeners, preservatives, sunscreens, dyes, perfumes, emulsifying agents, sterols, hydrating products, active products for the treatment of skin or scalp disorders and products for the regrowth of hair.

**11.** Composition according to Claims 9 or 10, characterised in that it is in the form of aqueous solution, of hydroalcoholic lotion, of oil-in-water or water-in-oil emulsion, of microemulsion, of microdispersion of lipid vesicles containing or not containing active products, of gel, of cake or of products for spraying.